# EUROPEAN PATENT APPLICATION

(11) **EP 4 717 264 A1**
(43) Date of publication of application: **01.04.2026**
(21) Application number: 25172071.0
(22) Date of filing: 23.04.2025
(51) Int. Cl.: A61K 31/352, A61P 29/00, A61P 17/06

(54) **TANGERETIN FOR USE IN THE TREATMENT OF INFLAMMATORY DISEASES**

(30) Priority: 30.09.2024 CN 202411383947
(71) Applicant: Xiangnan University, Chenzhou, Hunan 423099 (CN); Wang, Junjie, Chenzhou, Hunan 423099 (CN)
(72) Inventor: LIU, Qing, Chenzhou City, 423099 (CN); WANG, Junjie, Chenzhou City, 423099 (CN); LI, Yujuan, Chenzhou City, 423099 (CN); PENG, Qunlong, Chenzhou City, 423099 (CN); LIU, Siyu, Chenzhou City, 423099 (CN); LIU, Jing, Chenzhou City, 423099 (CN)
(74) Representative: Moré, Solveig Helga

(57) **Abstract**

The present disclosure relates to the technical field of biomedicine, in particular to use of an interleukin (IL)-17A inhibitor in the preparation of a medicament for treating an inflammatory disease. Use of an IL-17A inhibitor in the preparation of a medicament for treating an inflammatory disease is provided. Studies have proved that tangeretin (Tan) shows a significant anti-inflammatory activity both *in vivo* and *in vitro,* and may reduce inflammatory responses by inhibiting production of pro-inflammatory factors (such as tumor necrosis factor (TNF)-α and IL-6) and regulating IL-23/STAT3/IL-17A signaling pathways. Moreover, as a natural medicament, Tan may more safely and stably inhibit production of IL-17A and thus serves as a desirable IL-17A inhibitor.

## Description

### TECHNICAL FIELD

The present disclosure relates to the technical field of biomedicine, and in particular to use of an interleukin (IL)-17A inhibitor in the preparation of a medicament for treating an inflammatory disease.

### BACKGROUND

The interleukin-17 (IL-17) family, a subgroup of cytokines consisting of IL-17 (A to F), is produced by a variety of cells, including macrophages, Th17 cells, neutrophils, and natural killer (NK) cells. Since the discovery of IL-17A (also known as IL-17 or CTLA 8) in 1993, five other members of the family, IL-17B, IL-17C, IL-17D, IL-17E (also known as IL-25), and IL-17F, have been identified based on amino acid sequence homology. The most widely studied cytokine in this family, IL-17A, is a proinflammatory cytokine that plays an important role in host defense against microbial infections. This proinflammatory cytokine is also closely related to a variety of inflammatory diseases or autoimmune diseases, such as ankylosing spondylitis, multiple sclerosis, colitis, asthma, psoriasis, scleroderma, and rheumatoid arthritis. On one hand, IL-17A can induce the expression of cytokines related to tissue repair, thereby accelerating the body's recovery. On the other hand, IL-17A is highly expressed in a series of inflammatory diseases or autoimmune diseases. Moreover, since it can induce the accumulation of a large number of inflammatory factors, it leads to excessive immune responses. Elevated IL-17A levels may worsen the pathological development of related diseases. During the onset of various autoimmune or inflammatory diseases, an increased release of IL-23 further stimulates immune cells such as Th17 cells and macrophages (which are present in large numbers in various autoimmune and inflammatory diseases), activates the JAK-STAT3 signaling pathway, and stimulates these cells to release IL-17A after STAT3 phosphorylation. Subsequently, this process promotes the secretion of pro-inflammatory factors (such as tumor necrosis factor alpha (TNF-α) and IL-6) and pro-inflammatory mediators (such as nitric oxide, NO) in large numbers, thereby inducing local inflammatory reactions and then exacerbating the occurrence and development of a series of inflammatory or autoimmune diseases.

At present, significant research progress has been achieved in the development of IL-17A inhibitors, including Brodalumab, Bimekizumab, CJM112, Izokibep, and Secukinumab. These medicaments inhibit IL-17A or receptors thereof through different mechanisms of action to reduce inflammatory responses, showing desirable efficacy in the treatment of diseases closely related to IL-17A, such as ankylosing spondylitis, rheumatoid arthritis, and psoriasis. Meanwhile, IL-17A inhibitors have shown better efficacy and lower immunogenicity than traditional TNF-α inhibitors in clinical practice. Although biologic IL-17A inhibitors have significant efficacy as medicaments for treating a series of autoimmune diseases or inflammatory diseases such as ankylosing spondylitis, psoriasis, and rheumatoid arthritis, they generally exhibit a series of side effects such as gastrointestinal reactions, skin reactions, and respiratory infections. Therefore, finding and screening more efficient, safe, and stable anti-IL-17A medicaments is of great significance for the treatment of a series of autoimmune diseases or inflammatory diseases. In this context, natural medicaments with low toxicity and excellent biological activity provide a new path for the development of anti-IL-17A medicaments.

*Citri Reticulatae Pericarpium,* the dried tangerine peel is the mature fruit peel of the small tree plant *Citrus reticulate* Blanco and cultivated varieties thereof in the Rutaceae family. *Citri Reticulatae Pericarpium* contains many chemical components, mainly flavonoids, along with limonoids, alkaloids, and volatile oils. Existing pharmacological reports on *Citri Reticulatae Pericarpium* and its compounds highlight several effects: in the digestive system, it regulates gastrointestinal smooth muscle motility, promotes the secretion of digestive fluids and digestive enzyme, and protects liver. In the fight against respiratory diseases, the *Citri Reticulatae Pericarpium-derived* volatile oils exhibit antispasmodic and cough-relieving effects. Other reported pharmacological activities include positive inotropic effects, vasodilation and blood pressure reduction, anti-inflammatory, antioxidant, antiviral, antitumor, lipid-lowering, antibacterial, and immune-enhancing functions. The active ingredients of *Citri Reticulatae Pericarpium* show great potential in anti-inflammatory effects. Research by Li Xue et al. show that hesperidin can inhibit abnormal proliferation, impaired differentiation, and local inflammatory response of keratinocytes through the IRS-1/ERK1/2 signaling pathways. Research by Yang GuLiang et al. show that nobiletin can significantly inhibit the differentiation of CD4⁺T cells and the expression of transcription factors Ki-67 and PCNA. However, the anti-IL-17A effect in tangeretin (Tan), an active ingredient of the *Citri Reticulatae Pericarpium* has not yet been studied.

Tan is a natural flavonoid compound widely found in citrus peels. Related studies have shown that Tan has multiple biological activities such as anti-inflammatory (inhibiting the production of pro-inflammatory factors TNF-α and IL-6), antioxidant, neuroprotection, and anti-asthmatic effects. In addition, Tan also shows a great potential in the treatment of cancers including ovarian cancer, breast cancer, and gastric cancer.

Macrophages are important immune cells that are closely related to the initiation of inflammatory responses and are involved in a variety of inflammatory diseases or autoimmune diseases. These cells participate in immune responses through phagocytosis, antigen presentation, and cytokine secretion, playing a key role in the inflammatory response process. Lipopolysaccharide (LPS) is one of the main components of the cell wall of Gram-negative bacteria, and includes three parts of lipid A, core polysaccharide, and O-antigen. LPS can activate a variety of immune cells, such as macrophages, T lymphocytes, B lymphocytes, and NK cells. After treatment with LPS, macrophages shift to a pro-inflammatory state, resulting in a significant increase in inflammatory target mediators (such as NO) and inflammatory cytokines (such as TNF-α and IL-6), which can promote the inflammatory process. The LPS-induced RAW264.7 cellular inflammation model is an *in vitro* cell model widely used to study various diseases, including but not limited to autoimmune diseases such as psoriasis, rheumatoid arthritis, and systemic lupus erythematosus. The activation of macrophages and the production of inflammatory factors are closely related to the occurrence and development of these disease. For infectious diseases such as inflammatory responses caused by bacterial or viral infections, the RAW264.7 cell model can be used to study how pathogens activate immune cells and the host's immune response. For metabolic diseases, the model can be used, for example, to study the role of inflammatory responses in metabolic diseases such as obesity and diabetes. For neurodegenerative diseases such as Alzheimer's disease and Parkinson's disease, the model is useful in studying the role of inflammation in the neurodegenerative process. For tumors, the RAW264.7 cell model is also used to study the polarization state of macrophages in the tumor microenvironment and how they affect tumor growth and metastasis. Accordingly, in the study of inflammatory infiltration or immune inflammation, the LPS-induced RAW264.7 cellular inflammation model is a classic model for screening novel medicaments for the treatment of inflammatory diseases. Consequently, it is an urgent problem that those skilled need to solve to identify more types of medicaments for treating inflammatory diseases using the RAW264.7 cellular inflammation model to enrich the types of medicaments for treating inflammatory diseases in the prior art.

Psoriasis is an immune-mediated chronic inflammatory disease characterized by inflammatory responses mediated by a variety of immune cells (such as macrophages, T cells, and neutrophils) as well as abnormal proliferation and impaired differentiation of keratinocytes. IL-17A plays a central role in the pathogenesis of psoriasis. This factor not only affects the proliferation and cell function of keratinocytes, but also has an important regulatory effect on immune cells and related cytokines in the immunopathological environment of psoriasis. IL-17A promotes the activation and proliferation of keratinocytes by binding to an IL-17 receptor and stimulates the production of other proinflammatory cytokines and chemokines, leading to a positive feedback loop of inflammation within psoriasis lesions. Therefore, biological agents targeting IL-17A have shown significant efficacy in the treatment of psoriasis, and can effectively inhibit the occurrence of skin lesions and improve disease symptoms. In recent years, monoclonal antibodies targeting the IL-17A signaling pathway, such as secukinumab, ixekizumab, and brodalumab, have been launched in China and abroad and shown significant therapeutic effects in clinical applications. In addition, studies have also found that self-activation of the IL-17A signaling pathway can maintain inflammation and promote disease progression, providing a new research direction for the treatment of psoriasis. Targeting IL-17A presents a promising therapeutic strategy in the field of psoriasis treatment, offering more efficient and safe treatment options for patients. Since IL-17A plays an important role in the pathogenesis of psoriasis, *in vivo* animal experiments are conducted to establish a psoriasis mouse model using imiquimod (IMQ) to further clarify the *in vivo* anti-IL-17A activity of Tan after the anti-IL-17A effect of Tan is confirmed *in vitro.* After the anti-psoriatic effect of tangeretin is verified in IMQ-induced psoriasis mice, a tangeretin ointment preparation is administered to psoriasis patients for clinical use to further clarify the clinical efficacy.

Angiogenesis refers to the formation of new blood vessels from pre-existing ones, and occurs under a variety of physiological and pathological conditions, such as normal development, wound healing, menstrual cycles, and tumor growth. Angiogenesis is a complex biological process involving the interaction of multiple growth factors and signaling pathways, among which vascular endothelial growth factor (VEGF) is one of the most well-known angiogenesis-promoting factors. Angiogenesis is a pathological feature of psoriasis. The clinical pathogenesis of psoriasis mainly includes two major types of keratinocyte hyperproliferation and impaired differentiation and inflammatory cellular infiltration. With the hyperproliferation of keratinocytes, angiogenesis may inevitably increase. Therefore, angiogenesis is only an apparent pathological manifestation of psoriasis, and there is no direct connection between angiogenesis and the pathogenesis of psoriasis.

CN101947215A discloses use of a flavonoid compound derived from *Citri Reticulatae Pericarpium* in the preparation of a medicament for inhibiting angiogenesis. Endothelial cell proliferation assay and endothelial cell cycle assay were conducted using the 2,3-Bis-(2-methoxy-4-nitro-5-sulfophenyl)-2h-tetrazolium-5-carboxanilide (XTT) method, while zebrafish intersegmental and dorsal longitudinal vascular morphology were observed under a fluorescence confocal microscope system. The results show that the active ingredients in *Citri Reticulatae Pericarpium,* nobiletin, Tan, and sinensetin inhibit the proliferation of endothelial cells by regulating the cell cycle of endothelial cells, thereby inhibiting angiogenesis. The applicant thus inferred that the flavonoids in *Citri Reticulatae Pericarpium* could be used to prepare medicaments for treating diseases related to excessive angiogenesis. However, the applicant only conducted angiogenesis experiments, finding that Tan could significantly inhibit angiogenesis and cell proliferation, and speculated that the Tan may have certain anti-psoriasis activity. We acknowledge the experimental results and speculations of the applicant, and recognize that there is a connection between angiogenesis and psoriasis. Meanwhile, there may be deviations due to the different research fields and starting points. Retrospectively, the applicant's reasoning for linking angiogenesis to psoriasis seems to stem from the understanding that one of the pathogenesis of psoriasis is the abnormal proliferation and impaired differentiation of keratinocytes, which may lead to the pathological manifestation of angiogenesis. However, there is a lack of experimental data from psoriasis-related cell models or animal models to support this conclusion. Based on this, the "use of a flavonoid compound in *Citri Reticulatae Pericarpium* in the preparation of a medicament for treating psoriasis" in the patent is essentially unattainable and inaccurate.

### SUMMARY

An objective of the present disclosure is to provide use of an IL-17A inhibitor in the preparation of a medicament for treating an inflammatory disease. The present disclosure aims to develop a novel anti-IL-17A medicament, clarify the anti-inflammatory activity and anti-IL-17A effect of Tan, such that inflammatory responses can be inhibited and disease symptoms are improved. The present disclosure provides a novel treatment approach for a series of autoimmune diseases or inflammatory diseases such as ankylosing spondylitis, multiple sclerosis, colitis, asthma, scleroderma, and rheumatoid arthritis. In addition, as a natural medicament, Tan shows less toxicity and side effects, and thus is expected to solve a series of side effects caused by traditional IL-17A inhibitors. In general, the present disclosure aims to develop a novel anti-IL-17A medicament and clarify the anti-IL-17A effect of Tan to improve the physical and mental health and quality of life of patients suffering from diseases closely related to IL-17A.

To achieve the above objective, the present disclosure provides the following technical solutions.

The present disclosure provides use of an IL-17A inhibitor in the preparation of a medicament for treating an inflammatory disease, where the IL-17A inhibitor is Tan.

In some embodiments, the medicament further includes a pharmaceutically acceptable excipient.

In some embodiments, the medicament is selected from the group consisting of a tablet, a liquid, an ointment, a gel preparation, an emulsion, a capsule, a suppository, and a granule.

In some embodiments, the medicament for treating the inflammatory disease is selected from the group consisting of a medicament for treating ankylosing spondylitis, a medicament for treating multiple sclerosis, a medicament for treating colitis, a medicament for treating asthma, a medicament for treating psoriasis, a medicament for treating scleroderma, and a medicament for treating rheumatoid arthritis.

In some embodiments, the IL-17A inhibitor has a concentration of 5 µM to 40 µM in the medicament for treating the inflammatory disease.

In some embodiments, an inflammation-related factor of the inflammatory disease is selected from the group consisting of IL-23, IL-17A, TNF-α, and IL-6.

In some embodiments, the inflammatory disease is an LPS-induced inflammatory disease.

In some embodiments, the Tan has an effect of reducing a thickness of skin and a severity of scaling when the medicament for treating the inflammatory disease is the medicament for treating psoriasis.

In some embodiments, the Tan alleviates an inflammatory response by inhibiting production of pro-inflammatory factors and regulating IL-23/STAT3/IL-17A signaling pathways.

In some embodiments, the medicament for treating the inflammatory disease is a medicament for inhibiting the content of nitric oxide (NO), which is a cellular inflammatory factor.

Compared with the prior art, embodiments of the present disclosure has the following beneficial effects.

This study explored the anti-IL-17A effect of Tan in the LPS-induced RAW264.7 cellular inflammation model and the IMQ-induced psoriasis mouse model. It is found that in the LPS-induced RAW264.7 cellular inflammation model, Tan shows desirable anti-inflammatory activity and can effectively inhibit the production of IL-23 and IL-17A. This indicates that Tan can inhibit the inflammatory response by regulating the IL-23/IL-17A inflammatory axes and can be used as a novel IL-17A inhibitor. At the same time, Tan also provides a new path for the treatment of a series of autoimmune diseases or inflammatory diseases such as psoriasis, ankylosing spondylitis, colitis, scleroderma, and rheumatoid arthritis. Given that the IL-23/IL-17A inflammatory axes play an important role in a series of inflammatory diseases or autoimmune diseases, and in order to further clarify the mechanism of action of Tan in inhibiting IL-17A in macrophages, this study traced the upstream pathways of IL-17A secretion in cells, namely JAK/STAT3. Western-blot data show that the Tan treatment group in activated macrophages can significantly inhibit the phosphorylation of STAT3, and has a significant inhibitory effect on the phosphorylation of two STAT3 phosphorylation sites (Tyr 705 and Ser 727), with a more pronounced effect observed at Ser 727. This indicates that Tan may inhibit the secretion of IL-17A by inhibiting IL-23-mediated STAT3 phosphorylation. The above results also show that Tan exerts anti-inflammatory activity by regulating the IL-23/STAT3/IL-17A signaling pathways while inhibiting the production of IL-17A.

In the present disclosure, it is found in the IMQ-induced psoriasis mouse model that Tan can significantly improve the dorsal skin lesions of psoriasis mice and effectively reduce the back erythema and scaling. H&E staining results demonstrate that the Tan-treated mice exhibit a significant decrease in epidermal tissue thickness, a marked reduction in hyperkeratosis, and alleviation of inflammatory cellular infiltration in the dorsal skin, indicating that the anti-psoriatic activity of Tan is clearly dose-dependent. Immunohistochemistry shows that Tan can significantly inhibit the expression levels of p-STAT3 and IL-17A in the dorsal skin of psoriasis mice. This is consistent with the experimental results of an LPS-induced RAW264.7 cell validation model, highlighting the anti-IL-17A potential of Tan, and futher suggesting that Tan may exert anti-psoriatic activity by regulating the IL-23/STAT3/IL-17 signaling pathways. At the same time, immunohistochemistry results show that Tan can significantly inhibit the expression level of PCNA in the dorsal skin of mice, suggesting that Tan may combats psoriasis by inhibiting the abnormal proliferation and impaired differentiation of keratinocytes.

In the present disclosure, during the clinical application for psoriasis patients, a marked reduction in scaly erythematous plaques was observed after treatment. The scales almost completely disappeared after 10 days of administration. Patients treated with tangeretin ointment showed a significant decrease in overall Psoriasis Area and Severity Index (PASI) scores, demonstrating statistically significant differences compared to untreated body areas (p < 0.01), which confirms its remarkable anti-psoriatic efficacy.

For example, CN105582018A also discloses use of chikusetsu saponin IVa butyl ester as an inhibitor of IL-6/STAT3 signaling pathway. Diseases associated with the sustained activation of the IL-6/STAT3 signaling pathway include but are not limited to autoimmune diseases, inflammatory diseases, bone diseases, metabolic diseases, neurological and neurodegenerative diseases, cardiovascular diseases, allergies and asthma, Alzheimer's disease, and cancer. Based on the relevance of the pathway of the present disclosure to the prior art, Tan should also have the potential to prepare medicaments for treating the above diseases.

For example, CN117414379A discloses use of *Clinacanthus nutans* extract in the preparation of a medicament for preventing or treating ulcerative colitis. The *Clinacanthus nutans* extract inhibits the expression of proteins involved in inflammatory pathways, such as IKKβ/IKKα/NF-κB and JAK2/STAT3, and exerts a therapeutic effect on ulcerative colitis. Tan also works through these pathways, indicating its potential to treat colitis.

For example, CN114832053A discloses use of *Tetrastigma hemsleyanum* extract in the preparation of a medicament for treating rheumatoid arthritis. The *Tetrastigma hemsleyanum* extract regulates the IL- 6/STAT3/IL- 17 axes, modulates Th17 differentiation and Th17/Treg balance, and inhibits inflammatory response, representing new approach to treat RA. Tan also works through these pathways, indicating its potential to treat rheumatoid arthritis.

### BRIEF DESCRIPTION OF THE DRAWINGS

To describe the embodimets of the present disclosure or the technical solutions in the prior art more clearly, the drawings required for describing the embodiments or the prior art will be briefly described below. Apparently, the drawings in the following description merely illustrates embodiments of the present disclosure, and those of ordinary skill in the art can still derive other drawings from the provided drawings without creative efforts.
FIG. 1 shows a chemical structural formula of Tan;
FIGS. 2A-2B show effects of Tan and LPS on the viability of RAW264.7 cells; where FIG. 2A shows the effect of Tan on the viability of RAW264.7 cells; FIG. 2B shows the effect of Tan and LPS on the viability of RAW264.7 cells; compared with the Control group, * *P <* 0.05, ** *P* < 0.01, *** *P* < 0.001;
FIGS. 3A-3B show the effect of Tan on LPS-induced NO production in RAW264.7 cells; where FIG. 3A shows the effect of Tan on LPS-induced NO production in RAW264.7 cells (24 h), FIG. 3B shows the effect of Tan on LPS-induced NO production in RAW264.7 cells (48 h); compared with the Control group, ^{##} *P* < 0.001; compared with the LPS group, * *P* < 0.05, ** *P <* 0.01, *** *P* < 0.001;
FIG. 4A shows the effect of Tan on LPS-induced TNF-α secretion in RAW264.7 cells; FIG. 4B shows the effect of Tan on LPS-induced IL-6 secretion in RAW264.7 cells; where compared with the Control group, ^{##} *P* < 0.001; compared with the LPS group, * *P* < 0.05, ** *P <* 0.01, *** *P* < 0.001;
FIG. 5A shows the effect of Tan on LPS-induced IL-23 mRNA expression in RAW264.7 cells; FIG. 5B shows the effect of Tan on LPS-induced IL-17A mRNA expression in RAW264.7 cells; FIG. 5C shows the effect of Tan on LPS-induced TNF-α mRNA expression in RAW264.7 cells; FIG. 5D shows the effect of Tan on LPS-induced IL-6 mRNA expression in RAW264.7 cells; where compared with the Control group, ^{##} *P* < 0.001; compared with the LPS group, * *P <* 0.05, ** *P* < 0.01, *** *P* < 0.001;
FIG. 6A shows the expression levels of STAT3, p-STAT3 (Tyr 705), and p-STAT3 (Ser 727), as detected by Western blotting analysis; FIG. 6B, FIG. 6C, and FIG. 6D show semi-quantitative analysis of STAT3, p-STAT3 (Ser 727), and p-STAT3 (Tyr 705); where compared with the Control group, ^{##} *P* < 0.001; compared with the LPS group, * *P* < 0.05, ** *P <* 0.01, *** *P* < 0.001;
FIG. 7 shows representative photographs of the dorsal skin of mice in the Tan preventive administration group and the Tan therapeutic administration group;
FIG. 8 shows the H&E staining results of skin sections in the Tan preventive administration group and the Tan therapeutic administration group, scale bar = 100 µm, magnification = 100×;
FIG. 9 shows the expression level of p-STAT3 in the dorsal skin of mice in the Tan preventive administration group and the Tan therapeutic administration group, scale bar = 200 µm, magnification = 200×;
FIG. 10 shows the expression level of IL-17A in the dorsal skin of mice in the Tan preventive administration group and the Tan therapeutic administration group, scale bar = 200 µm, magnification = 200×;
FIG. 11 shows the expression level of PCNA in the dorsal skin of mice in the Tan preventive administration group and the Tan therapeutic administration group, scale bar = 200 µm, magnification = 200×;
FIGS. 12A-12H show a comparison of a patient with severe psoriasis before and after treatment with Tan ointment: arm scales are significantly reduced and erythema is subsided; where FIG. 12A shows conditions before treatment, FIG. 12B to FIG. 12H show conditions after treatment, and FIG. 12H shows conditions of 14 d after treatment;
FIG. 13 shows the results of PASI scoring of psoriasis patients during treatment, where the PASI scores of psoriasis patients are greatly reduced after treatment with the Tan ointment preparation; compared with day 0, ** *P* <0.05, **** P* < 0.01, **** *P* < 0.001; and
FIG. 14 shows a technology roadmap of the present disclosure.

### DETAILED DESCRIPTION OF THE EMBODIMENTS

The technical solutions provided by the present disclosure will be described in detail below with reference to examples, but the examples should not be construed as limiting the claimed scope of the present disclosure.

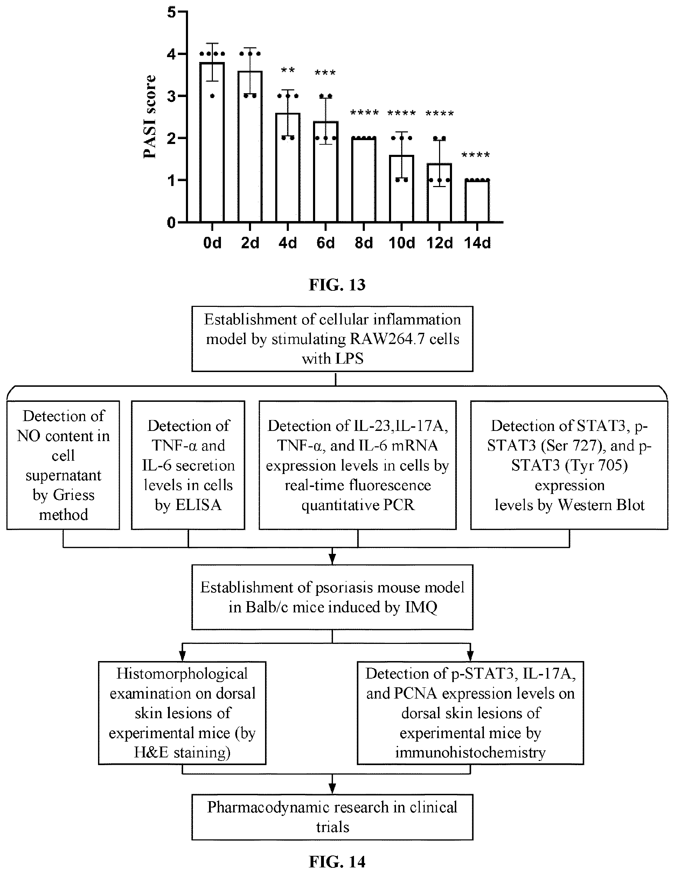

In the present disclosure, RAW264.7 cells are stimulated with LPS to establish a cellular inflammation model and an IMQ-induced psoriasis mouse model to systematically explore the anti-inflammatory activity and mechanism of action of Tan. The technology roadmap is shown in FIG. 14. The cellular inflammation model is established by stimulating RAW264.7 cells with LPS and the psoriasis mouse model is established using IMQ to study the anti-inflammatory activity and mechanism of action of Tan. Clinical trials are conducted to evaluate the anti-psoriasis pharmacodynamics of Tan.

RAW264.7 cell culture: mouse mononuclear macrophages (RAW264.7, ATCC) are cultured in a complete medium containing 10% fetal bovine serum (FBS), 1% double antibodies (penicillin and streptomycin), and 89% DMEM at 37°C and 5% CO₂. In this experiment, RAW264.7 cells from passages 3 to 8 are used for related test.

LPS (100 ng/mL) is added for stimulation, with or without Tan, and the cell supernatant is collected to detect the contents of NO, IL-23, IL-17A, TNF-α, and IL-6 in the cell supernatant. The cell lysate is collected and the protein expressions (STAT3, p-STAT3 (Ser727), and p-STAT3 (Tyr705)) are detected by Western blot. Total cell RNA is extracted and real-time fluorescence quantitative polymerase chain reaction (PCR) is conducted to detect the expression levels of IL-23, IL-17A, TNF-α, and IL-6 mRNA.

Statistics: the experimental data are analyzed using Excel 2021 and GraphPad Prism 9.5 software, and the experimental results are expressed as Mean ± SD values. The comparison between multiple groups is conducted with the student's T test (two-tailed) for statistical analysis, where *P* < 0.05 is considered statistically significant, *P* < 0.01 is considered statistically significant, and *P* < 0.001 is considered extremely significant.

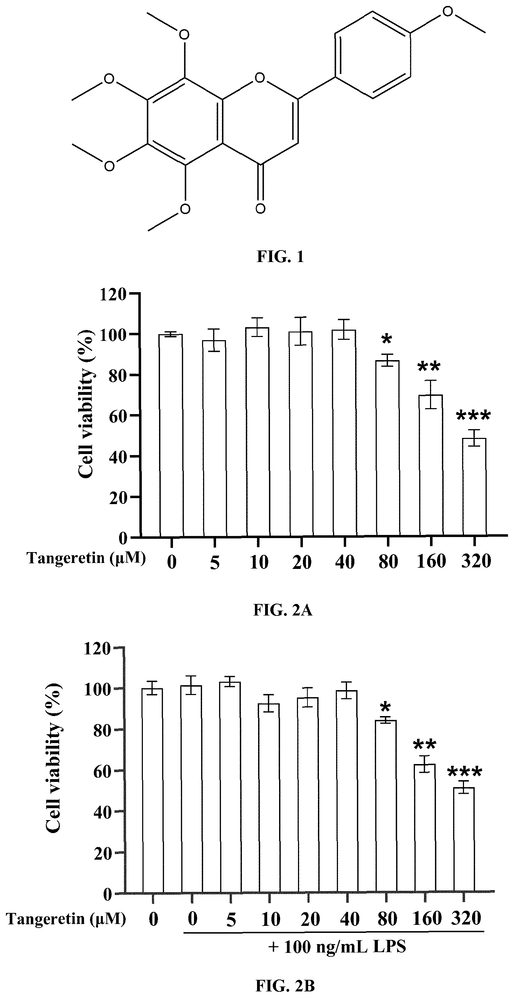

**Experimental medicaments and reagents:** Tan (purity ≥98.0%, chemical structure shown in FIG. 1) is purchased from Shanghai Yuanye Biotech Co., Ltd., and dissolved in dimethyl sulfoxide (DMSO) (MeilunBio, cell culture-grade) to prepare a 100 mM stock solution. Preparation of tangeretin ointment (tangeretin vaseline ointment) in clinical trials includes the following steps: 9.616 g of Vaseline is added into an evaporating dish, heated in a water bath to allow melting, 0.384 g of tangeretin powder is added while stirring, stirred well, and cooled to allow solidification).

LPS and clobetasol propionate (Clo) are purchased from Shanghai Aladdin Biochemical Technology Co., Ltd., and dissolved in DMSO to prepare 1 mg/mL and 50 mM stock solutions, respectively. IMQ cream (Mingxinlidi), Clo cream (Fuyuan Pharmaceutical) are adopted. Cell proliferation and toxicity detection (CCK-8) kit and Bicinchoninic Acid (BCA) quantitative kit (MeilunBio^{®}); NO detection kit (Beyotime^{®}); Enzyme-linked immunosorbant assay (ELISA) (TNF-α and IL-6) kit (Lianke Biotechnology); RNA extraction kit (Jianshi Biotech); reverse transcription kit and real-time fluorescence quantitative PCR kit (abm^{®}); protein lysis buffer (Solarbio^{®}); GAPDH and STAT3 antibodies (Proteintech Group, Inc); p-STAT3, IL-17A, p-STAT3 (Ser 727), and p-STAT3 (Tyr 705) antibodies (CST) are adopted.

Experimental instruments include: cell culture incubator (Thermo^{®}), centrifuge (Eppendorf^{®}), analytical balance (SartoroiusStedim Biotech), microplate reader (BioTek^{®}), gene amplifier (Dongsheng International Trading Company), real-time fluorescence quantitative PCR instrument (Thermo^{®}), vertical electrophoresis instrument and protein transfer system (BioRad^{®}), gel imaging system (SinSage Technology Co., Ltd.).

### Example 1 Cytotoxic effects of Tan and LPS on RAW264.7 cells detected by CCK-8 method

RAW264.7 cells in the logarithmic growth phase were inoculated into a 96-well plate and the cell density was adjusted to 5×10³ cells/well. After cultured for 24 h, the cells were stably adhered to the wall. The original complete medium was removed and different concentrations of Tan (5, 10, 20, 40, 80, 160, 320 µM) were added to allow treatment with or without LPS (100 ng/mL) for 24 h. Then, 10 µL of CCK-8 solution was added to each well and incubated at 37°C for 1 h. The OD value of each sample at 450 nm was detected by a microplate reader, and the cell viability was calculated.

(Cell viability (%) = [A (medicament administration) - A (blank)] / [A (0 medicament administration) - A (blank)] × 100%, NOTE: A (medicament administration): OD value of wells with cells, CCK-8 solution, and medicament solution; A (0 medicament administration): OD value of wells with cells and CCK-8 solution without medicament; A (blank): OD value of wells without cells.)

### Results: effects of Tan and LPS on RAW264.7 cell viability

The CCK-8 results showed (FIG. 2A to FIG. 2B) that there was no statistically significant difference for the effect of LPS (100 ng/mL) on the viability of RAW264.7 cells (P>0.05). Tan did not produce significant toxicity to RAW264.7 cells at a concentration of 0-40 µM for 24 h *(P* > 0.05), but the cell viability of RAW264.7 cells decreased when the Tan concentration was higher than 40 µM *(P* < 0.05). Since the subsequent experiments were conducted to study the anti-inflammatory activity and anti-IL-17A effect of Tan in the RAW264.7 cellular inflammation model, it was necessary to treat cells with the medicament at a non-cytotoxic concentration. Therefore, the subsequent experimental concentration of Tan was determined to be 0-40 µM, and the subsequent experimental concentration of LPS was determined to be 100 ng/mL.

### Example 2 Effect of Tan on LPS-induced NO production in RAW264.7 cells detected by Griess method

RAW264.7 cells in the logarithmic growth phase were inoculated into a 48-well plate and the cell density was adjusted to 1.0×10⁵ cells/well. After cultured for 24 h, the cells were stably adhered to the plate. The original medium was removed and different concentrations (5, 10, 20, 40 µM) of Tan were added to allow pretreatment for 1 h. LPS (100 ng/mL) was added and incubated for a further 24 h, and the cell supernatant was collected. OD_{540 nm} value of the cell supernatant was detected according to the instructions of a Griess kit, and the NO content in the cell supernatant was calculated.

### Results: effect of Tan on LPS-induced NO production in RAW264.7 cells

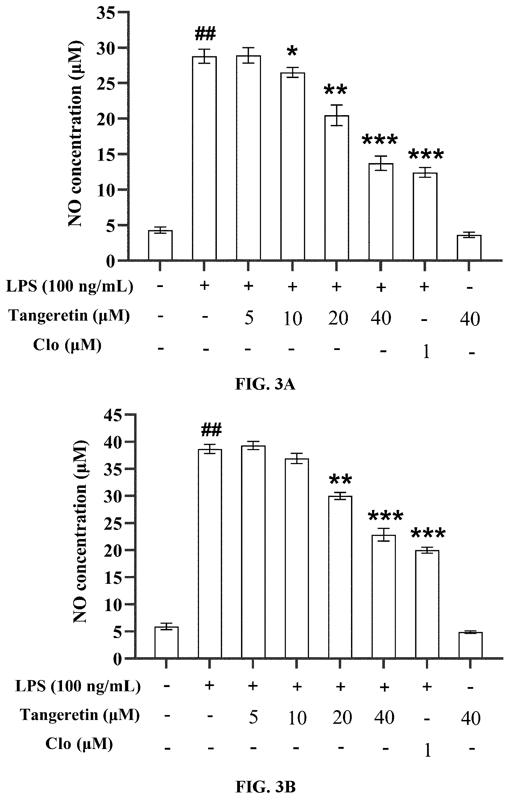

Griess results showed (FIG. 3A to FIG. 3B) that compared with that of the Control group, the NO content in the cell supernatant of the LPS group was significantly increased (*P* < 0.001), indicating that LPS stimulated RAW264.7 cells to successfully establish a cellular inflammation model. The NO content in the cell supernatant of the group treated with Tan for 24 h was significantly reduced (*P* < 0.001) in a dose-dependent manner, and the inhibitory effect of Tan on NO at a concentration of 40 µM was comparable to that of the positive control, which was treated with clobetasol propionate (Clo). In the group treated with Tan for 48 h, the NO content in the cell supernatant of each group increased overall, but Tan still showed a strong inhibitory effect on the production of NO by RAW264.7 cells. It was preliminarily determined that Tan had a certain anti-inflammatory activity.

### Example 3 Effect of Tan on LPS-induced pro-inflammatory cytokine secretion levels in RAW264.7 cells detected by ELISA

RAW264.7 cells in the logarithmic growth phase were inoculated into a 6-well plate and the cell density was adjusted to 5.0×10⁵ cells/well. After culturing for 24 h, the cells were stably adhered to the plate. The original medium was removed and different concentrations (5, 10, 20, 40 µM) of Tan were added to allow pretreatment for 1 h. LPS (100 ng/mL) was added and incubated for a further 24 h, and the cell supernatant was collected. The OD_{450 nm} value of the cell supernatant was detected according to the instructions of the TNF-α and IL-6 ELISA kits, and the content of pro-inflammatory factors (TNF-α and IL-6) in the cell supernatant was calculated.

### Results: effect of Tan on LPS-induced pro-inflammatory cytokine secretion in RAW264.7 cells

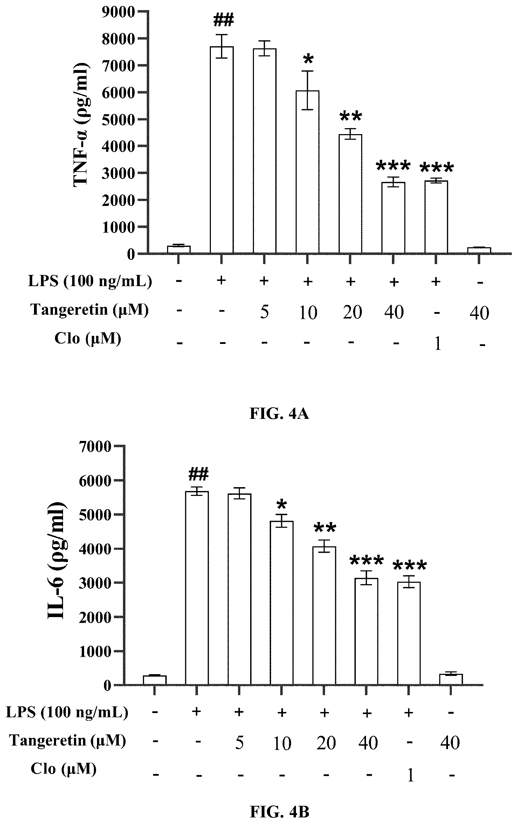

ELISA results showed (FIG. 4A to FIG. 4B) that compared with those of the Control group, the levels of proinflammatory factors (TNF-α and IL-6) in the cell supernatant of the LPS group were significantly increased (*P* < 0.001). After treatment with Tan, the levels of proinflammatory factors (TNF-α and IL-6) in the cell supernatant were significantly decreased *(P* < 0.001) in a dose-dependent manner. The inhibitory effect of Tan on the secretion of proinflammatory factors (TNF-α and IL-6) at a concentration of 40 µM was comparable to that of the positive control, which was treated with clobetasol propionate (Clo). This indicated that Tan could dose-dependently inhibit the production of proinflammatory factors in RAW264.7 cells and thus reduce the inflammatory response.

### Example 4 Effect of Tan on LPS-induced IL-23, IL-17A, TNF-α, and IL-6 mRNA expressions in RAW264.7 cells detected by real-time fluorescence quantitative PCR

RAW264.7 cells were inoculated into 6-well plate and the cell density was adjusted to 5.0×10⁶ cells/well. After cultured for 24 h, the cells were stably adhered to the wall. The original medium was removed and different concentrations (5, 10, 20, 40 µM) of Tan were added to allow pretreatment for 1 h. LPS (100 ng/mL) was added and continued to be incubated for 24 h. The commercial TRlcom lysis buffer provided by Jianshi Biotech was added to extract total RNA from cells using the centrifugal column method and immediately reverse transcribed into c-DNA. The fluorescence intensity of each sample was detected according to the instructions of the real-time fluorescence quantitative PCR kit and specific primers (Table 1) (cycle conditions: enzyme activation 95°C for 3 min, 1 cycle; denaturation 95°C for 15 s, annealing/extension 60°C for 1 min, 40 cycles). The mRNA expression level of each group was normalized with the GAPDH expression level, and the relative expression of mRNA was calculated using the 2^{-ΔΔCt} method.

**Table 1 Real-time fluorescence quantitative PCR system**

| Component | Volume |
|---|---|
| BlasTaq^{™}2×qPCR MM¹ | 10 µL |
| Forward Primer(10 µM) | 0.5 µL |
| Reverse Primer(10 µM) | 0.5 µL |
| Template DNA | 100 ng |
| Nuclease-free H₂O | up to 20 µL |

**Table 2 Primer sequences for real-time fluorescence quantitative PCR**

| Gene | Primer sequences(5'-3')-F | Primer sequences(5'-3')-R | Length |
|---|---|---|---|
| GAPDH | AGGTCGGTGTGAACGGATTTG (SEQ ID NO: 1) | TGTAGACCATGTAGTTGAGGTCA (SEQ ID NO: 2) | 123 bp |
| IL-23 | CTCGGTGAACAACTGAGGGA (SEQ ID NO: 3) | GGTGGAATCTCTGCCCACTT (SEQ ID NO: 4) | 90 bp |
| IL-17A | ACTACCTCAACCGTTCCACG (SEQ ID NO: 5) | TTCCTCCGCATTGACACAG (SEQ ID NO: 6) | 120 bp |
| TNF-α | ATGAGCACAGAAAGCATGATC (SEQ ID NO: 7) | GGTCTGGGCCATAGAACTGATG (SEQ ID NO: 8) | 231 bp |
| IL-6 | TAGTCCTTCCTACCCCAATTTCC (SEQ ID NO: 9) | TTGGTCCTTAGCCACTCCTTC (SEQ ID NO: 10) | 76 bp |

The sequences are shown in SEQ ID NO: 1 to SEQ ID NO: 10.

### Results: effect of Tan on LPS-induced IL-23, IL-17A, TNF-α, and IL-6 mRNA expressions in RAW264.7 cells

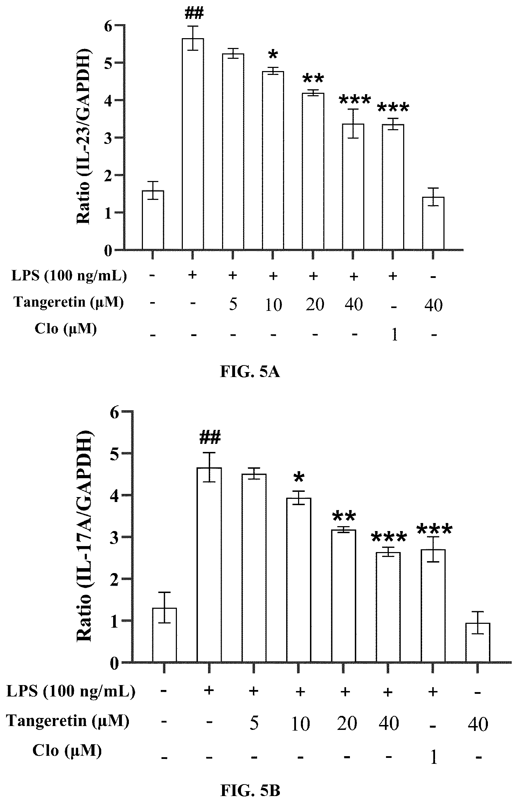

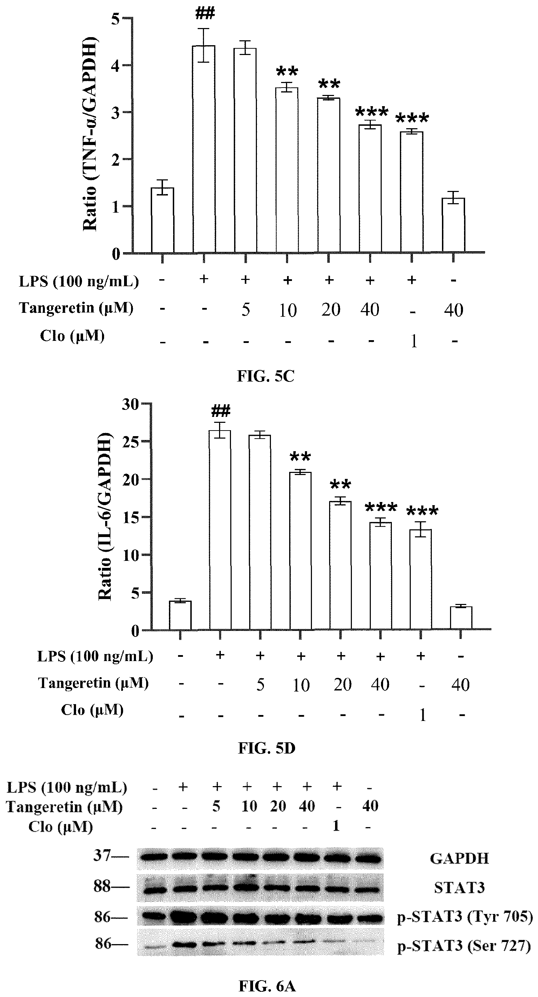

The results of real-time fluorescence quantitative PCR showed (FIG. 5A to FIG. 5D) that the relative expression levels of IL-23, IL-17A, TNF-α, and IL-6 mRNA were significantly increased after LPS stimulation compared with those of the Control group (*P <* 0.001). After Tan treatment, the relative expression levels of IL-23, IL-17A, TNF-α, and IL-6 mRNA in the experimental cells were significantly decreased (*P* < 0.001) in a dose-dependent manner. Moreover, the inhibitory effect of Tan at a concentration of 40 µM on the mRNA of proinflammatory factors (IL-23, IL-17A, TNF-α, and IL-6) was comparable to that of the positive control Clo. The experimental results (FIG. 5B) showed that Tan exhibited a strong inhibitory effect on IL-17A, and Tan inhibited the inflammatory response by regulating the IL-23/IL-17A inflammatory axes.

### Example 5 Effect of Tan on LPS-induced STAT3 protein phosphorylation level in RAW264.7 cells

RAW264.7 cells in the logarithmic growth phase were inoculated in 100 mm culture dishes, and the cell density was adjusted to 1.6×10⁶ cells/dish. After cultured for 24 h, different concentrations (5, 10, 20, 40 µM) of Tan were added to allow pretreatment for 2 h, and LPS (100 ng/mL) was added to allow incubation for 24 h. The original medium was removed, and the cells were washed 2 times with pre-cooled PBS buffer. Then, protein lysis buffer (300 µL per dish) was added for lysis on ice for 30 min. The supernatant was collected by centrifugation at 4°C, 12,000 rpm for 5 min, and the protein concentration was determined by BCA method. Next, 60 µg of total protein sample was loaded, separated by 10% Sodium Dodecyl Sulfate Polyacrylamide Gel Electrophoresis (SDS-PAGE) gel, and transferred to Polyvinylidene Fluoride (PVDF) membrane by electrophoresis. The membrane was blocked with 5% skim milk at room temperature for 1 h, and 3 mL of GAPDH, STAT3, p-STAT3 (Ser727), and p-STAT3 (Tyr705) antibodies were added and incubated overnight at 4°C. Afterwards, 5 mL of horseradish peroxidase-labeled secondary antibody was added and incubated at room temperature for 1 h. Enhanced Chemiluminescence (ECL) color development was used for detection, and protein band quantification was performed using Image J software.

### Results: effect of Tan on LPS-induced STAT3 protein phosphorylation level in RAW264.7 cells

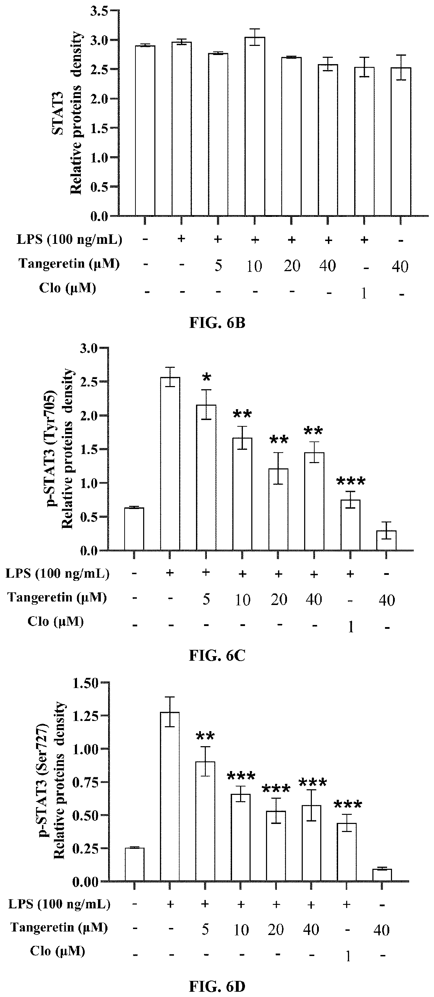

Western blot results showed (FIG. 6A to FIG. 6D) that there was no statistically significant change in STAT3 protein expression among the treatment groups (*P*>0.05). In terms of STAT3 protein phosphorylation, compared with the Control group, the LPS group demonstrated significantly increased phosphorylation of STAT3 at both sites (Tyr 705 and Ser 727) (*P* < 0.001). *(P* < 0.001). Compared with the LPS group, the Tan treatment group inhibited the expression of p-STAT3 (Tyr 705) and p-STAT3 (Ser 727) proteins in a dose-dependent manner (*P* < 0.001), with a more pronounced suppression observed for p-STAT3 (Ser 727) than p-STAT3 (Tyr 705). At 40 µM, tangeretin exhibited comparable efficacy to the positive control clobetasol propionate (Clo) in suppressing STAT3 phosphorylation. This further support that Tan mitigates the inflammatory response by regulating the IL-23/STAT3/IL-17A signaling pathways.

### Example 6 Effect of Tan on dorsal skin lesions of IMQ-induced psoriasis mice

Balb/c mice (female, 6-8 weeks, 20±2 g), with certificate number [SCXK (Xiang) 2021-0002], were purchased from Hunan SJA Laboratory Animal (Changsha, China). All mice were housed in an specific pathogen free (SPF)-grade animal room with a 12 h light/12 h dark cycle, and experiments were started after the mice were acclimated for one week. Next, 96 mice were randomly divided into 12 groups (8 mice/group), including Control group, Control solvent (white vaseline) group, IMQ group, IMQ solvent (white vaseline) group, preventive administration of positive medicament Clo group (IMQ+Clo 55 mg), therapeutic administration of positive medicament group (IMQ+Clo (A) 55 mg), preventive administration of Tan low-dose group (IMQ+Tan 40 µg/cm²), medium-dose group (IMQ+Tan160 µg/cm²), and high-dose group (IMQ+Tan 640 µg/cm²), and therapeutic administration of Tan low-dose group (IMQ+Tan (A) 40 µg/cm²), medium-dose group (IMQ+Tan (A) 160 µg/cm²), and high-dose group (IMQ+Tan (A) 640 µg/cm²). After the mice were anesthetized, a 2 cm×3 cm area on the back was selected for hair removal. Modeling began 48 h after hair removal, and all modeling mice were applied with 65 mg (55 mg on the back and 5 mg on each ear) of IMQ cream every morning for 10 d. In the Tan preventive administration groups, the low-dose group (40 µg/cm²), the medium-dose group (160 µg/cm²), and the high-dose group (640 µg/cm²) were topically administered to the back and ears every afternoon starting from the first day of modeling for 10 d. In the Tan therapeutic administration groups, the low-dose group (40 µg/cm²), the medium-dose group (160 µg/cm²), and the high-dose group (640 µg/cm²) were topically administered to the back and ears every afternoon starting from the 3rd day of modeling for 10 d. The mice in the preventive positive medicament group received Clo (55 mg/mouse, 45 mg on the back, 5 mg on each ear) treatment from the first day of modeling, and the mice in the therapeutic positive medicament group received Clo (55 mg/mouse, 45 mg on the back, 5 mg on each ear) treatment from the third day of modeling.

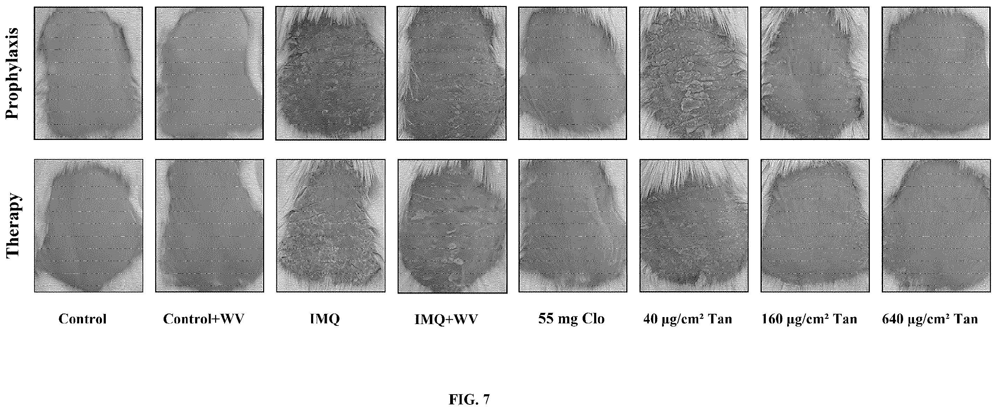

Results: Tan significantly inhibited the dorsal skin lesions of psoriasis mice
As shown in FIG. 7, the mice in the IMQ group had significant erythema and scaling on the back compared with the mice in the Control group. Compared with the IMQ group, the Tan preventive and therapeutic administration groups had a significant effect on improving the pathological symptoms of psoriasis, with a significant reduction in erythema and scaling in a dose-dependent manner. The improvement effect of the high-dose Tan preventive and therapeutic groups on the dorsal skin lesions of psoriasis mice was comparable to that of the positive control Clo. The scales and erythema on the dorsal skin of the mice in the preventive group almost disappeared, and the skin condition was similar to that of the Control group. After treatment with the therapeutic group, the skin pathological symptoms of psoriasis mice were greatly improved, with only a minimal amount of back scales remained. The above research results all showed that Tan could reduce skin thickness and the severity of scales (FIG. 7).

### Example 7 Histological evaluation of dorsal skin lesions of IMQ-induced psoriasis mice by Tan

The dorsal skin tissues of mice were fixated in 4% paraformaldehyde solution for 30 d and then stained with hematoxylin and eosin (H&E). The detailed operation steps were as follows. The fixated tissue was placed in ethanol solutions with graded concentrations for dehydration, and then transparentized in xylene for 30 min each time. The sample was embedded in paraffin to make serial sections with 3-5 µm thickness. After dewaxed and rehydrated, the sections were stained with hematoxylin-eosin reagent to identify the skin tissue structure and cell nuclei, and then dried and sealed with neutral resin. Images were collected using an optical microscope (DM500, Leica^{®}, Germany) at a magnification of 100 times.

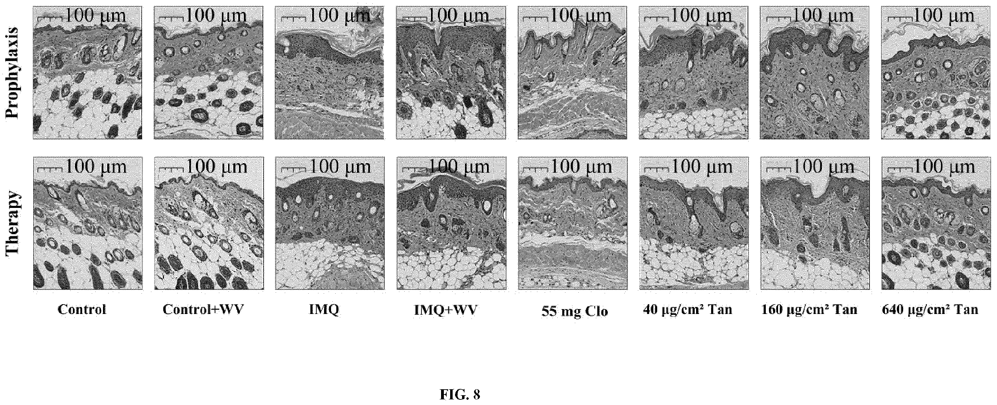

Results: Tan significantly improved the thickening and impaired differentiation of keratinocytes and inflammatory cellular infiltration in the dorsal skin of psoriasis mice
Compared with the Control group, the IMQ group had obvious epidermal tissue thickening, parakeratosis, and massive inflammatory cellular infiltration. Compared with the IMQ group, the preventive Tan group showed reduced epidermal tissue thickness, reduced hyperkeratosis, and slight inflammatory cellular infiltration in the dorsal skin of mice, and the activity was obviously dose-dependent. Compared with the IMQ group, the therapeutic Tan group also showed stronger pharmacological activity, especially in the high-dose group, which showed reduced epidermal tissue thickness, reduced hyperkeratosis, and slight inflammatory cellular infiltration, also in a significant dose-dependent manner. In summary, the results of H&E staining of mouse skin lesions showed that Tan exhibited strong anti-psoriasis activity regardless of preventive or therapeutic administration, and there was a significant dose-dependency (FIG. 8).

### Example 8 Effect of Tan on the expression levels of p-STAT3, IL-17A, and PCNA in the dorsal skin of IMQ-induced psoriasis mice

The dorsal skin samples of mice were collected and made into paraffin sections, which were then dewaxed with xylene, and incubated in H₂O₂ solution at room temperature for 10 min to inhibit the activity of endogenous peroxidase. The sections were blocked with BSA at room temperature for 1 h to remove nonspecific background adsorption. The p-STAT3, IL-17A, and PCNA antibodies were added and incubated overnight at 4°C. Horseradish peroxidase-labeled secondary antibodies were added and incubated at room temperature for 30 min. 3,3'-Diaminobenzidine (DAB) was added for color development and hematoxylin was used for counterstaining. A negative control was set up and photographed with unified parameters. The magnification was 200 times for observation.

Results: Tan significantly inhibited the expression levels of p-STAT3, IL-17A, and PCNA in the dorsal skin of psoriasis mice

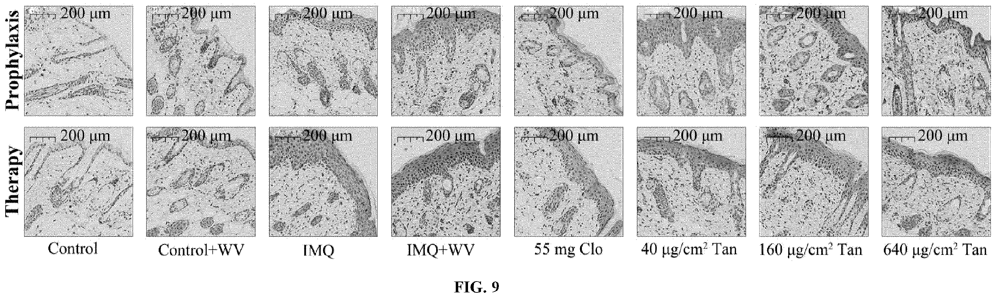

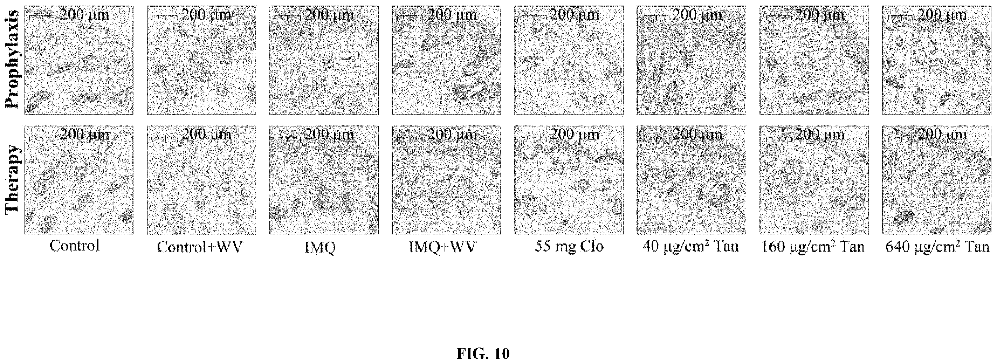

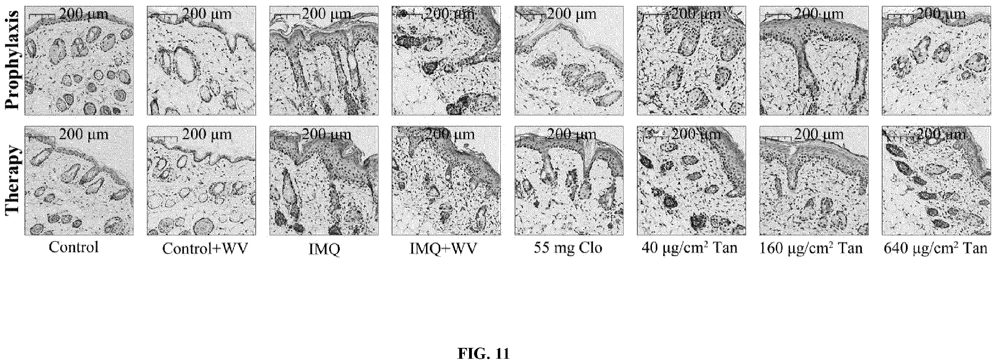

Compared with those of the Control group, the expression levels of p-STAT3, IL-17A, and PCNA in the IMQ group were significantly increased (FIG. 9, FIG. 10, and FIG. 11), and the expression levels of p-STAT3, IL-17A, and PCNA in the dorsal skin of mice in the Tan preventive and therapeutic administration groups were significantly reduced in a dose-dependent manner. This indicated that Tan could significantly inhibit the production of p-STAT3, IL-17A, and PCNA, thereby reducing inflammatory responses and abnormal proliferation and impaired differentiation of keratinocytes to exert anti-psoriasis activity. Tan was thus an excellent anti-IL-17A inhibitor.

In summary, the above studies show that Tan has significant anti-inflammatory activity, and may reduce inflammatory responses by inhibiting production of pro-inflammatory factors (such as TNF-α and IL-6) and regulating IL-23/STAT3/IL-17A signaling pathways. Moreover, as a natural medicament, Tan can more safely and stably inhibit the production of IL-17A and thus serves as a desirable IL-17A inhibitor.

### Example 9 Typical treatment case of Tan ointment preparation for psoriasis patients

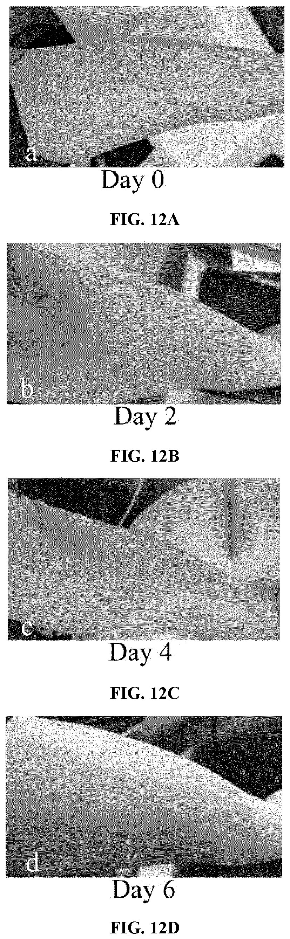

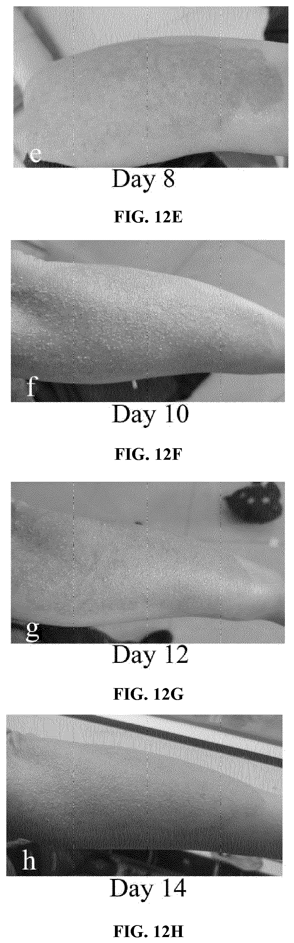

A 35-years-old male patient, Ning, developed scaly erythema on his trunk after catching a cold 15 years ago, accompanied by itching. He went to the local hospital and was diagnosed with psoriasis vulgaris. He used Clo, retinoic acid, and calcipotriol for external treatment. The rash recurred and gradually spread to his trunk and limbs. The psoriasis lesions on the whole body involved more than 50% of the skin, and current clinical diagnosis demonstrated severe psoriasis vulgaris. The Tan ointment of the present disclosure was used for topical application treatment once a day (the single use of the ointment was 6 mg/cm², and the dosage was 230 µg/cm²). After 4 d of treatment, the symptoms improved significantly. After 10 d of continued administration, the symptoms improved significantly and the scales almost completely disappeared (FIG. 12). To ensure the privacy of the patient, the patient's identity was not disclosed here, and the patient's identity information and case were archived in the Affiliated Hospital of Xiangnan College and were available for reference.

Results: Tan significantly improved the symptoms of scaly erythema on the arm skin of psoriasis patients.

According to FIG. 12A-FIG. 12H, Mr. Ning, the patient with psoriasis, had extremely thick and numerous scales on his arm. After the administration of Tan ointment for 2 d, the scales began to decrease. After the administration for 4 d, the scales on the patient's arm decreased significantly and almost disappeared completely. The continued administration of the medicament had a more pronounced effect. On the 10th day after administration of the medicament, the scales had completely disappeared. On the 14th day, the color of the erythema on the affected skin area became significantly lighter, the erythema lesions significantly shrank and became smaller, and healthy skin appeared.

### Example 10 Clinical trial on the efficacy of Tan ointment preparation

Five patients with clinically confirmed psoriasis were observed, and Tan ointment preparation was applied topically to the affected area once a day for 14 d. The day before Tan ointment preparation treatment was defined as day 0, and the day of the first administration of Tan ointment was defined as day 1. During this period, patients were monitored for the Psoriasis Area and Severity Index (PASI) to assess the therapeutic effect of Tan on psoriasis. The severity scoring of skin lesions included parameters such as erythema and scaling, with each parameter rated from 0 to 4 (0 indicating no clinical symptoms, no scaling, and no erythema; 1 indicating mild clinical symptoms with slight erythema; 2 indicating moderate clinical symptoms with mild erythema and scaling; 3 indicating significant clinical symptoms with pronounced erythema and extensive scaling; and 4 indicating very severe clinical symptoms). During the treatment process, it was observed that after the application of the Tan ointment preparation (applied to the affected area once daily at a dosage of 6 mg/cm², with an amount of 230 µg/cm²), psoriasis patients exhibited significant improvement by day 4. By days 9-10, the scaling had almost completely disappeared, and continued administration resulted in a lightening of the skin redness and a marked reduction in symptoms. The PASI monitoring data indicated a significant decrease in overall PASI scores following treatment with the Tan ointment preparation, demonstrating a very pronounced anti-psoriatic effect (FIG. 13).

Result: Tan significantly improved the pathological symptoms of psoriasis patients, and the PASI scores of psoriasis patients were significantly reduced after treated with the Tan ointment preparation.

According to the results in FIG. 13, after the 5 psoriasis patients used the Tan ointment preparation, their PASI scores began to decrease after the 2nd day of medication, and then significantly reduced after the 4th day of medication. With the extension of the treatment duration, the PASI scores continued to decrease, indicating that the pathological symptoms of psoriasis patients in the experimental group showed significant improvement.

The above descriptions are merely preferred embodiments of the present disclosure. It should be noted that a person of ordinary skill in the art may further make several improvements and modifications without departing from the principle of the present disclosure, but such improvements and modifications should be deemed as falling within the protection scope of the present disclosure.

## Claims

1. An interleukin (IL)-17A inhibitor for use in treating an inflammatory disease, wherein the IL-17A inhibitor is tangeretin (Tan).

2. The IL-17A inhibitor for use according to claim 1, wherein the interleukin (IL)-17A inhibitor is in the form of a medicament, and the medicament further comprises a pharmaceutically acceptable excipient.

3. The IL-17A inhibitor for use according to claim 2, wherein the medicament is selected from the group consisting of a tablet, a liquid, an ointment, a gel preparation, an emulsion, a capsule, a suppository, and a granule.

4. The IL-17A inhibitor for use according to claim 2, wherein the medicament is selected from the group consisting of a medicament for treating psoriasis, a medicament for treating ankylosing spondylitis, a medicament for treating multiple sclerosis, a medicament for treating colitis, a medicament for treating asthma, a medicament for treating scleroderma, and a medicament for treating rheumatoid arthritis.

5. The IL-17A inhibitor for use according to claim 2, wherein the IL-17A inhibitor has a concentration of 5 µM to 40 µM in the medicament.

6. The IL-17A inhibitor for use according to claim 1, wherein an inflammation-related factor of the inflammatory disease is selected from the group consisting of IL-23, IL-17A, tumor necrosis factor (TNF)-α, and IL-6.

7. The IL-17A inhibitor for use according to claim 1, wherein the inflammatory disease is a lipopolysaccharide (LPS)-induced inflammatory disease.

8. The IL-17A inhibitor for use according to claim 4, wherein the Tan reduces skin thickness and scaling severity when the medicament is the medicament for treating psoriasis.

9. The IL-17A inhibitor for use according to claim 1, wherein the Tan alleviates an inflammatory response by inhibiting production of pro-inflammatory factors and regulating IL-23/STAT3/IL-17A signaling pathways.

10. The IL-17A inhibitor for use according to claim 2, wherein the medicament is a medicament for inhibiting the content of nitric oxide (NO), which is a cellular inflammatory factor.
